Europäisches Patentamt

European Patent Office

Office européen des brevets

(19)

(11)  EP 0 838 210 A2

(12)  **EUROPÄISCHE PATENTANMELDUNG**

(43) Veröffentlichungstag:
29.04.1998  Patentblatt 1998/18

(51) Int. Cl.⁶: **A61K 7/06**

(21) Anmeldenummer: 97110977.2

(22) Anmeldetag: 02.07.1997

(84) Benannte Vertragsstaaten:
AT BE CH DE DK ES FI FR GB GR IE IT LI LU MC
NL PT SE
Benannte Erstreckungsstaaten:
AL LT LV RO SI

(30) Priorität: 28.09.1996 DE 19640099

(71) Anmelder:
Wella Aktiengesellschaft
64274 Darmstadt (DE)

(72) Erfinder:
• Titze, Hans-Jürgen
64401 Gross-Bieberau (DE)
• Steinbrecht, Karin, Dr.
64372 Ober-Ramstadt (DE)
• Birkel, Susanne, Dr.
64380 Rossdorf (DE)
• Franzke, Michael, Dr.
64380 Rossdorf-Gundernhausen (DE)

(54) **Verwendung von wasserunlöslichen Fasern in kosmetischen Mitteln zur Haarbehandlung**

(57)  Gegenstand der Erfindung ist die Verwendung mindestens einer wasserunlöslichen synthetischen oder natürlichen Faserart oder deren Gemische zur Herstellung eines kosmetischen Mittels zur Behandlung, Gestaltung oder Pflege der Frisur.

Mit dem erfindungsgemäßen Mittel behandeltes Haar erhält im trockenen Zustand eine gute Festigung und eine große Spannkraft bei einem gleichzeitig natürlichen Griff und seidigen Glanz.

EP 0 838 210 A2

**Beschreibung**

Gegenstand der vorliegenden Erfindung ist die Verwendung von mindestens einer wasserunlöslichen synthetischen oder natürlichen Faserart oder deren Gemische zur Herstellung eines kosmetischen Mittels zur Behandlung, Gestaltung oder Pflege der Frisur.

Ein ansprechendes äußeres Erscheinungsbild wurde schon immer als sehr wichtig angesehen. Eine besondere Rolle spielt dabei die Frisur. Basis für ein ansprechendes Äußeres ist gut frisiertes und gepflegtes Haar. Für die Reinigung und Pflege des Haares gibt es eine ganze Reihe von Haarbehandlungsmitteln wie Shampoos, Kuren, Spülungen, Spitzenfluids, die in den unterschiedlichsten Anwendungsarten, zum Beispiel als leave on- oder als rinse-off-Produkte, appliziert werden. Neben diesen Pflegeprodukten kommen drei weitere Produktkategorien bei der Haarveränderung zum Einsatz, nämlich permanente oder temporäre Haarfärbemittel, permanente Haarverformungsmittel in Form von mildalkalischen oder sauren Dauerwellen beziehungsweise Haarentkrausungsmitteln sowie Mittel, die nur eine temporäre Verformung und Stabilisierung der Frisur ermöglichen und allgemein als Stylingmittel bekannt sind. Hierzu zählen Produkte wie Haarsprays, Haarlacke, Festigerlotionen, Festigerschäume, Haargele, glanzgebende Produkte, Frisurcremes etc. Allen diesen Mitteln ist gemeinsam, daß sie in der Regel aus einer Vielzahl an Einzelsubstanzen oder Komponenten bestehen, die die unterschiedlichsten Aufgaben innerhalb der Rezeptur erfüllen.

Gesucht werden daher Substanzen, die in möglichst vielen Rezepturen verträglich sind. Zu den Forderungen, die an gut frisiertes Haar gestellt werden, zählt beispielsweise, daß das Haar im trockenen Zustand einen angenehmen Griff, Elastizität, Volumen (Ausnahme "Wetlook" Frisuren) und Halt haben sollte. Kurz gesagt, das Haar sollte sich nach Haar anfühlen.

Die Aufgabe dieser Erfindung bestand also darin, eine neue Substanzklasse zu finden, die die oben genannten Anforderungen erfüllt. Eine solche Substanzklasse wurde mit der im folgenden näher erläuterten Substanzklasse der wasserunlöslichen Fasern gefunden.

Gegenstand der vorliegenden Erfindung ist daher die Verwendung von mindestens einer wasserunlöslichen synthetischen oder natürlichen Faserart oder deren Gemische zur Behandlung, Gestaltung oder Pflege der Frisur sowie zur Herstellung eines kosmetischen Mittels zur Behandlung, Gestaltung oder Pflege der Frisur.

Synthetische und natürliche Fasern sind in einer Vielzahl von Haarbehandlungsmitteln einsetzbar.

Künstliche, synthetische Fasern sind dreidimensionale, längliche, haarähnliche Polymere oder natürliche Polymere mit einer eindeutig definierten chemischen Struktur.

Einsetzbare Fasern können synthetisch, wie zum Beispiel Viskose-, Polyester- oder Polyamidfasern oder natürlich, wie zum Beispiel Seiden-, Cellulose-, Flachs-, Leinen-, Schafwoll- oder Baumwollfaser sein.

Die künstlichen Fasern zeichnen sich durch ihre glatte Faserstruktur und gute mechanische Eigenschaften, zum Beispiel Reißfestigkeit, Biegesteifigkeit etc., aus.

Geeignete Polyamidfasern sind beispielsweise (Poly)hexamethylenadipamid) (Nylon 6.6, Polyamid 6.6), Poly($\varepsilon$-Caprolactam) (Polyamid 6), Polyamid 6.12 oder Polyamid 11.12. Besonders bevorzugt ist Poly(hexamethylenadipamid). Die Polyamidfasern weisen vorzugsweise eine Länge von 150 bis 2000 $\mu$m auf.

Die klassischen Polyesterfasern sind seidenähnlich. Typische, geeignete Polymere sind vor allem Polyethylenterephthalat. Auch sogenannte Mikrofasern bestehen aus Polyester. Viskosefasern oder auch Rayonfasern werden durch chemische Modifikationsverfahren aus Cellulose hergestellt. Auch diese Viskosefasern können in den erfindungsgemäßen Mitteln eingesetzt werden.

Von den natürlichen Fasern ist die Seidenfaser die hier bevorzugte. Die Seidenfaser ist aus Proteinen aufgebaut, (wie zum Beispiel Serin, Glycin, Alanin, Glutaminsäure, Threonin, etc.). Sie zeichnet sich durch ihre glatte, glänzende Oberfläche aus. Dadurch, daß sie sich an das Haar anlagert, verstärkt sie das Haar und verhilft ihm auch zu Glanz. Eine hervorragende Eigenschaft ist das Wasserrückhaltevermögen, welches cirka 42 Prozent ist, wobei die Feuchtigkeitsaufnahme cirka 11 Prozent beträgt, was der des Haares nahekommt. Die Seidenfaser weist vorzugsweise eine Länge von 1 bis 1100 $\mu$m auf.

Mischungen der oben genannten synthetischen mit natürlichen Fasern in verschiedenen Verhältnissen können ebenfalls eingesetzt werden.

Die Fasern zeichnen sich dadurch aus, daß sie eine haarähnliche Struktur haben, insbesondere die natürlichen Fasern. Sie legen sich an das Haar an und verstärken dadurch den Halt der Frisur. Dies wurde durch Messungen der Bruchkraft bestätigt. Die Fasern haben vorzugsweise einen Durchmesser von 8 $\mu$m bis 70 $\mu$m, besonders bevorzugt von 10 $\mu$m bis 30 $\mu$m. Die Länge liegt vorzugsweise bei 20 $\mu$m bis 2000 $\mu$m, besonders bevorzugt bei 150 $\mu$m bis 750 $\mu$m.

Die Fasern werden bevorzugt in einer Menge von 0,01 bis 2,5 Gewichtsprozent, besonders bevorzugt in einer Menge von 0,05 bis 1 Gewichtsprozent des kosmetischen Mittels eingesetzt. Das erfindungsgemäße Mittel kann beispielsweise in Form von Haarfestigungs-, Haarfärbe-, Tönungs- oder Blondiermitteln vorliegen. Das Mittel kann als Lotion, Schaum, Milch, Gel, Creme, Gelschaum, Spray appliziert werden.

Das erfindungsgemäße Mittel kann darüber hinaus die für Haarbehandlungsmittel üblichen Zusatzbestandteile enthalten, zum Beispiel Lösungsmittel, wie Wasser und niedere aliphatische Alkohole, beispielsweise Ethanol, Propanol und Isopropanol, oder Glykole wie Glycerin und 1,2-Propylenglykol; Netzmittel oder Emulgatoren aus den Klassen der anionischen, kationischen, amphoteren oder nicht-ionogenen oberflächenaktiven Tenside, wie Fettalkoholsulfate, Alkylbenzolsulfonate, Alkyltrimethylammoniumsalze, Feuchthaltemittel, Alkylbetaine, oxethylierte Fettalkohole, oxethylierte Nonylphenole, Fettsäurealkanolamide, oxethylierte Fettsäureester, in einer Menge von 0,1 bis 30 Gewichtsprozent, Parfümöle in einer Menge von 0,1 bis 0,5 Gewichtsprozent; Trübungsmittel, wie zum Beispiel Ethylenglykoldistearat, in einer Menge von etwa 0,2 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10 Gewichtsprozent; bakterizide und fungizide Wirkstoffe; wie zum Beispiel 2,4,4-Trichlor-2-hydroxydiphenylether oder Methylchlorisothiazolin, in einer Menge von 0,01 bis 1,0 Gewichtsprozent; Verdickungsmittel, wie beispielsweise Kokosfettsäurediethanolamid, in einer Menge von etwa 0,2 bis 3,0 Gewichtsprozent, Puffersubstanzen, wie beispielsweise Natriumcitrat oder Natriumphosphat, in einer Menge von 0,1 bis 1,0 Gewichtsprozent; Lösungsvermittler, wie zum Beispiel ethoxyliertes Rizinusöl, in einer Menge von etwa 0,1 bis 1,0 Gewichtsprozent; Anfärbestoffe, wie zum Beispiel Fluorescein-Natriumsalz, in einer Menge von etwa 0,1 bis 1,0 Gewichtsprozent; Pflegestoffe, wie zum Beispiel Pflanzen- und Kräuterextrakte, Protein- und Seidenhydrolysate, kationische Harze, Lanolinderivate, in einer Menge von 0,1 bis 5 Gewichtsprozent; physiologisch verträgliche Silikonderivate, wie zum Beispiel Silikonöl, Silikonpolymere und Siloxane; Lichtschutzmittel, Antioxidantien, Radikalfänger, Antischuppenwirkstoffe, in einer Menge von etwa 0,01 bis 2 Gewichtsprozent; physiologisch verträgliche organische Säuren, wie zum Beispiel Ameisensäure, Glyoxylsäure, Milchsäure, Weinsäure, Zitronensäure, natürliche, modifizierte natürliche oder synthetische Polymere, wie zum Beispiel Schellack, kationische, anionische, nichtionische, amphotere Polymere, Hydroxyalkylcellulose, Chitosan, Chitin oder Chitosanderivate; direktziehende Haarfarbstoffe, Haarfarbstoffe, die oxidativ entwickelt werden, Oxidationsmittel, Fettalkohole, Glanzgeber, Vitamine, Weichmacher, Kämmbarkeitsverbesserer, rückfettende Agentien, Entschäumer sowie Treibgase, wie zum Beispiel Fluorkohlenwasserstoffe, Dimethylether, Kohlenwasserstoffe und komprimierbare Gase.

Aufgrund der Verstärkung der festigenden Eigenschaften durch den Faserzusatz in Haarstylingprodukten hält die Frisur länger und besser.

In einer bevorzugten Ausführungsform enthält das erfindungsgemäße Mittel zusätzlich mindestens ein natürliches und/oder synthetisches Polymer, welches ausgewählt ist aus den Gruppen der festigenden und der verdickenden Polymere. Die Polymere können in Mengen von 0,01 bis 25 Gewichtsprozent, bevorzugt 0,1 bis 20 Gewichtsprozent, eingesetzt werden und in gelöster Form oder als Dispersion vorliegen. Ein derartiges Mittel kann bei Einsatz von festigenden Polymeren mit Faserzusatz als Haarfestigungsmittel verwendet werden.

Das erfindungsgemäße kosmetische Mittel kann unter Verwendung eines Treibmittels oder mit Hilfe einer mechanisch betriebenen Sprühvorrichtung versprüht werden oder mit Hilfe einer Schaumerzeugungsvorrichtung als Schaum abgegeben werden. Hierzu werden spezielle Ventile eingesetzt.

Wenn das erfindungsgemäße kosmetische Mittel mit Hilfe eines Treibmittels versprüht wird, so enthält es bevorzugt 3 bis 75 Gewichtsprozent des Treibmittels und wird in einen Druckbehälter abgefüllt.

Als Treibmittel sind beispielsweise niedere Alkane, wie zum Beispiel n-Butan, i-Butan und Propan oder deren Gemische oder auch Dimethylether und Fluorkohlenwasserstoff, wie beispielsweise F 152 (1,1-Difluorethan) oder F 134 (Tetrafluorethan) sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Unter mechanischen Sprühvorrichtungen oder Schaumerzeugungsvorrichtungen sind solche Vorrichtungen zu verstehen, welche das Versprühen oder Aufschäumen einer Flüssigkeit ohne Verwendung eines Treibmittels ermöglichen. Als geeignete mechanische Sprühvorrichtung kann beispielsweise eine Sprühpumpe für Pulver oder ein mit einem Sprühventil versehener elastischer Behälter, in den das erfindungsgemäße kosmetische Mittel unter Druck abgefüllt wird, wobei sich der elastische Behälter ausdehnt und aus dem Mittel infolge der Kontraktion des elastischen Behälters beim Öffnen des Sprühventils kontinuierlich abgegeben wird, verwendet werden.

Wird das erfindungsgemäße kosmetische Mittel zur Haarfestigung eingesetzt, so wird es folgendermaßen angewandt:

Nach der Haarwäsche werden in dem handtuchtrockenen Haar, je nach Haarfülle, 5 bis 30 g des Mittels verteilt. Anschließend wird das Haar durchgekämmt und zur Frisur geformt und getrocknet.

Das erfindungsgemäße Mittel kann zum Beispiel ein Flüssigfestiger, Schaumfestiger, Haargel oder auch ein Haarspray sein. Nach der Anwendung des oben genannten Mittels wird mehr Halt aufgrund der verstärkenden Wirkung der Fasern im Haar festgestellt.

Ein erfindungsgemäßes Haartönungsmittel enthält 0,05 bis 3 Gewichtsprozent gefärbte oder farblose Fasern und zusätzlich 0,05 bis 2,0 Gewichtsprozent mindestens eines direkt auf das Haar aufziehenden Haarfarbstoffs, der beispielsweise aus den folgenden Klassen direkt auf das Haar aufziehender Haarfarbstoffe ausgewählt sein kann: aromatische Nitrofarbstoffe, zum Beispiel 1,4-Diamino-2-nitrobenzol, Azofarbstoffe, zum Beispiel Acid Brown 4 (C. I. 14 805), Anthrachinonfarbstoffe, zum Beispiel Disperse Violet 4 (C. I. 61 105), Triphenylmethanfarbstoffe, zum Beispiel Basic Violet 1 (C. I. 42 535), wobei die Farbstoffe je nach Art ihrer Substituenten sauren, nichtionischen oder basischen Charakter haben können oder eines natürlichen Haarfarbstoffs, wie zum Beispiel Henna oder Reng, der zur Farbentwicklung nicht der Oxidation bedarf.

Mit dem erfindungsgemäßen Mittel behandeltes Haar erhält im trockenen Zustand eine gute Festigung und eine große Sprungkraft bei einem gleichzeitig natürlichen Griff und seidigen Glanz.

Die nachfolgenden Beispiele sollen den Gegenstand der Erfindung näher erläutern.

**Beispiele**

Es werden die folgenden Fasern eingesetzt:

| | |
|---|---|
| Seidenfaser (1): | Seidenfaser mit einer Faserlänge zwischen 1 und 160 μm (Crosilk Powder der Firma Croda/Deutschland) |
| Seidenfaser (2): | Seidenfaser mit einer Faserlänge zwischen 200 und 720 μm (vertrieben durch die Firma Interorgana/Deutschland) |
| Seidenfaser (3): | Seidenfaser mit einer Faserlänge zwischen 260 und 1100 μm (vertrieben durch die Firma Interorgana/Deutschland) |
| Polyamidfaser: | Poly(hexamethylenadipamid) (Nylon 6.6) mit einer Faserlänge zwischen 150 und 2000 μm (vertrieben durch die Firma STW Schwarzwälder Textilwerke/Deutschland) |
| Viskosefaser: | Viskosefaser mit einer Faserlänge zwischen 150 und 2000 μm (vertrieben durch die Firma REO Flock & Faser/Deutschland) |

**Beispiel 1:** Haarbalsam

| | |
|---|---|
| 0,50 g | Seidenfaser (1) |
| 6,00 g | Glycerylstearat/Polyethylenglykol-(20)-cetearylether |
| 4,00 g | Diquaternäres Polydimethylsiloxan (Abil® Quat 3272 der Firma Goldschmidt/Deutschland) |
| 2,00 g | Cetylalkohol |
| 1,36 g | Zitronensäure |
| 0,14 g | 1,2-Dibrom-2,4-dicyanobutan |
| 0,12 g | Parfüm |
| 85,88 g | Wasser |
| 100,00 g | |

**Beispiel 2:** Haarspülung

| | |
|---|---|
| 0,75 g | Polyamidfaser |

| | |
|---|---|
| 4,00 g | Cetylstearylalkohol |
| 1,36 g | DL-2-Pyrrolidon-5-carbonsäure |
| 0,75 g | Cetyltrimethylammoniumchlorid |
| 0,50 g | Parfüm |
| 0,20 g | Pflanzenextrakt Extrapon® 5 Spezial der Firma Dragoco/Deutschland |
| 92,44 g | Wasser |
| 100,00 g | |

**Beispiel 3:** Versprühbares Haarkurmittel

| | |
|---|---|
| 0,15 g | Seidenfaser (1) |
| 2,00 g | Dimethyldiallylammoniumchlorid |
| 1,25 g | Polyethylenglykol-(40)-sorbitanmonopalmitat |
| 1,00 g | DL-2-Pyrrolidon-5-carbonsäure |
| 0,10 g | Parfüm |
| 0,03 g | Cetyltrimethylammoniumchlorid |
| 15,15 g | Ethanol |
| 80,32 g | Wasser |
| 100,00 g | |

**Beispiel 4:** Schaumförmiges Haarkurmittel

| | |
|---|---|
| 0,40 g | Seidenfaser (2) |
| 2,00 g | Kationische Emulsion von aminfunktionalisiertem Polydimethylsiloxan (929 Cationic Emulsion der Firma Dow Corning Europe/Belgien) |
| 1,30 g | Zitronensäure |
| 0,50 g | Hydroxypropylcellulose (M = 1.150.000 g/mol) |
| 0,30 g | Silikonwachs (2501 Cosmetic Wax der Firma Dow Corning Europe/Belgien) |
| 0,20 g | Parfüm |
| 0,25 g | Cetyltrimethylammoniumchlorid |
| 0,15 g | D-Panthenol |
| 0,10 g | Elastinhydrolysat |
| 5,00 g | Propan/Butan (5,0 bar) |
| 10,00 g | Ethanol |
| 79,80 g | Wasser |
| 100,00 g | |

**Beispiel 5:** Haarfestigungsmittel

| | |
|---|---|
| 0,10 g | Seidenfaser (3) |
| 3,00 g | Vinylpyrrolidon/Vinylacetat Copolymer |
| 0,90 g | Ameisensäure |
| 0,20 g | 1,2-Propylenglykol |
| 0,15 g | Parfüm |
| 0,03 g | Cetyltrimethylammoniumchlorid |
| 20,20 g | Wasser |
| 75,42 g | Ethanol |
| 100,00 g | |

**Beispiel 6:** Haarfestigungsmittel

| | |
|---|---|
| 0,25 g | Polyamidfaser |

| | |
|---|---|
| 0,25 g | Seidenfaser (2) |
| 3,00 g | Vinylpyrrolidon/Vinylacetat Copolymer |
| 0,90 g | Ameisensäure |
| 0,20 g | 1,2-Propylenglykol |
| 0,15 g | Parfüm |
| 0,03 g | Cetyltrimethylammoniumchlorid |
| 20,00 g | Wasser |
| 75,22 g | Ethanol |
| 100,00 g | |

**Beispiel 7:** Haarfestigungsmittel

| | |
|---|---|
| 0,20 g | Seidenfaser (1) |
| 3,00 g | Vinylpyrrolidon/Vinylacetat Copolymer |
| 0,90 g | Ameisensäure |
| 0,20 g | 1,2-Propylenglykol |
| 0,15 g | Parfüm |
| 0,03 g | Cetyltrimethylammoniumchlorid |
| 20,30 g | Wasser |
| 75,22 g | Ethanol |
| 100,00 g | |

**Beispiel 8:** Haarfestigungsmittel

| | |
|---|---|
| 0,10 g | Viskosefaser |
| 3,00 g | Vinylpyrrolidon/Vinylacetat Copolymer |
| 0,90 g | Ameisensäure |
| 0,20 g | 1,2-Propylenglykol |
| 0,15 g | Parfüm |
| 0,03 g | Cetyltrimethylammoniumchlorid |
| 20,20 g | Wasser |
| 75,42 g | Ethanol |
| 100,00 g | |

**Beispiel 9:** Fönlotion mit UV-Schutz

| | |
|---|---|
| 0,15 g | Seidenfaser (2) |
| 2,15 g | DL-2-Pyrrolidon-5-carbonsäure |
| 1,50 g | Vinylpyrrolidon/Vinylacetat Copolymer |
| 1,25 g | Vinylpyrrolidon/Dimethylaminoethylmethacrylat Copolymer |
| 0,20 g | Parfüm |
| 0,15 g | Glycerin (85prozentig) |
| 0,10 g | 2-Hydroxy-4-methoxybenzophenon |
| 42,90 g | Wasser |
| 51,60 g | Ethanol |
| 100,0 g | |

**Beispiel 10:** Haarpflegende Festigerlotion

| | |
|---|---|
| 0,25 g | Seidenfaser (3) |
| 4,00 g | Vinylpyrrolidon/Vinylacetat Copolymer |
| 1,20 g | DL-2-Pyrrolidon-5-carbonsäure |
| 0,40 g | Hydriertes Rizinusöl, ethoxyliert mit 40 Mol Ethylenoxid |
| 0,20 g | Parfüm |
| 93,95 g | Wasser |
| 100,00 g | |

**Beispiel 11:** Schaumfestiger mit starker Festigung

| | |
|---|---|
| 0,2 g | Polyamidfaser |
| 5,00 g | Vinylpyrrolidon/Vinylacetat Copolymer |
| 0,60 g | DL-2-Pyrrolidon-5-carbonsäure |
| 0,45 g | Glyceryllaurat |
| 0,15 g | Parfüm |
| 0,06 g | Cetyltrimethylammoniumchlorid |
| 5,00 g | Propan/Butan (5,0 bar) |
| 10,40 g | Ethanol |
| 77,74 g | Wasser |
| 100,00 g | |

**Beispiel 12:** Schaumfestiger

| | |
|---|---|
| 0,10 g | Seidenfaser (3) |
| 1,80 g | Chitosan |
| 1,10 g | Ameisensäure |
| 0,20 g | 1,2-Propylenglykol |
| 0,20 g | Parfüm |
| 0,10 g | Cetyltrimethylammoniumchlorid |
| 6,00 g | Propan/Butan (5,0 bar) |
| 10,20 g | Ethanol |
| 80,30 g | Wasser |
| 100,00 g | |

**Beispiel 13:** Schaumfestiger

| | |
|---|---|
| 0,25 g | Viskosefaser |
| 3,15 g | Polyvinylpyrrolidon |
| 1,60 g | Zitronensäure |
| 0,60 g | Hydriertes Rizinusöl, ethoxyliert mit 40 Mol Ethylenoxid |
| 0,22 g | Decylpolyglucosid |
| 0,20 g | Vinylpyrrolidon/Methacrylamidopropyltrimethylammoniumchlorid |
| 0,20 g | Parfüm |
| 6,00 g | Propan/Butan (5,0 bar) |
| 87,78 g | Wasser |
| 100,00 g | |

**Beispiel 14:** Lockenschaumfestiger

| | |
|---|---|
| 0,05 g | Seidenfaser (2) |
| 2,20 g | Vinylimidazoliummethochlorid/1-Vinyl-2-Pyrrolidon-Copolymer |
| 1,00 g | Glucosesirup |
| 0,70 g | Oleyl-polyethylenglykol-(200)-ether |
| 0,60 g | Zitronensäure |
| 0,10 g | Parfüm |
| 7,00 g | Propan/Butan (5,0 bar) |
| 10,20 g | Ethanol |
| 78,15 g | Wasser |
| 100,00 g | |

**Beispiel 15:** Pflegender Schaumfestiger

| | |
|---|---|
| 0,2 g | Seidenfaser (3) |

| | |
|---|---|
| 6,0 g | Vinylcaprolactam/Vinylpyrrolidon/Dimethylaminoethylmethacrylat Terpolymer |
| 0,6 g | Ameisensäure |
| 0,2 g | Cetyltrimethylammoniumchlorid |
| 0,2 g | Hydriertes Rizinusöl, ethoxyliert mit 45 Mol Ethylenoxid |
| 0,2 g | Parfüm |
| 96,0 g | Wasser |
| 100,0 g | |

Die Mischung wird im Verhältnis 94:6 mit einem Propan/Butan-Treibgasgemisch abgefüllt.

**Beispiel 16:** Farbfestiger

| | |
|---|---|
| 0,23 g | Seidenfaser (1) |
| 2,50 g | Vinylacetat/Crotonsäure/Polyglykol Copolymer |
| 0,20 g | Parfüm |
| 0,07 g | 1-Amino-4-(2',3'-dehydroxypropyl)amino-5-chlor-2-nitrobenzol |
| 0,05 g | Basic Brown 17 (C. I. 12 251) |
| 0,01 g | Basic Blue 7 (C. I. 42 595) |
| 0,0023 g | Basic Violett 14 (C. I. 42 510) |
| 46,94 g | Wasser |
| 50,00 g | Ethanol |
| 100,0 g | |

**Beispiel 17:** Haarspray mit starker Festigung

| | |
|---|---|
| 0,15 g | Seidenfaser (3) |
| 5,00 g | t-Octylacrylamid/Acrylsäure/t-Butylaminoethylmethacrylat Terpolymer |
| 0,58 g | 2-Amino-2-methyl-1-propanol |
| 0,15 g | Parfüm |
| 40,00 g | Propan/Butan 1.5 |
| 54,12 g | Ethanol |
| 100,00 g | |

**Beispiel 18:** Haarspray

| | |
|---|---|
| 0,05 g | Polyamidfaser |
| 3,50 g | Vinylacetat/Crotonsäure/Vinylneodecanoat Terpolymer |
| 0,15 g | Parfüm |
| 0,14 g | Ameisensäure |
| 45,00 g | Dimethylether |
| 51,16 g | Ethanol |
| 100,00 g | |

**Beispiel 19:** Styling-Haarspray

| | |
|---|---|
| 0,10 g | Seidenfaser (2) |
| 6,50 g | Vinylpyrrolidon/Vinylacetat Copolymer |
| 0,17 g | Ameisensäure |
| 0,10 g | Parfüm |
| 10,67 g | Butan (1,5 bar) |
| 33,33 g | Propan/Butan |

| | |
|---|---|
| 43,13 g | Ethanol |
| 100,00 g | |

**Beispiel 20:** 80 % VOC-Haarspray

| | |
|---|---|
| 0,10 g | Polyamidfaser |
| 0,10 g | Seidenfaser (3) |
| 4,00 g | t-Butylacrylat/Ethylacrylat/Methacrylsäure Terpolymer |
| 0,72 g | 2-Amino-2-methyl-1-propanol |
| 0,20 g | Cyclo-Tetra(dimethylsiloxan) |
| 0,05 g | Parfüm |
| 15,00 g | Wasser |
| 39,83 g | Ethanol |
| 40,00 g | Dimethylether |
| 100,00 g | |

**Beispiel 21:** Pumpspray

| | |
|---|---|
| 0,05 g | Polyamidfaser |
| 4,50 g | t-Octylacrylamid/Acrylsäure/t-Butylaminoethylmethacrylat Terpolymer |
| 0,52 g | 2-Amino-2-methyl-1-propanol |
| 0,30 g | Parfüm |
| 0,10 g | Dimethylsiloxan/Ethylenglykol Copolymer |
| 6,53 g | Wasser |
| 88,00 g | Ethanol |
| 100,00 g | |

**Beispiel 22:** 80 % VOC-Pumpspray

| | |
|---|---|
| 0,075 g | Seidenfaser (1) |
| 5,00 g | Vinylpyrrolidon/Vinylacetat Copolymer |
| 0,30 g | Parfüm |
| 0,26 g | Ameisensäure |
| 13,84 g | Wasser |
| 80,525 g | Ethanol |
| 100,000 g | |

**Beispiel 23:** 55 % VOC-Pumpspray

| | |
|---|---|
| 0,10 g | Viskosefaser |
| 3,50 g | Vinylacetat/Crotonsäure Copolymer |
| 0,28 g | Ameisensäure |
| 0,20 g | Parfüm |
| 40,23 g | Wasser |
| 55,69 g | Ethanol |
| 100,00 g | |

**Beispiel 24:** Haargel

| | |
|---|---|
| 0,20 g | Seidenfaser (1) |
| 2,50 g | Hydroxypropylmethylcellulose |
| 0,80 g | Polyoxyethylen-(20)-sorbitanmonopalmitat |
| 0,50 g | Polyoxyethylen-(25)-p-aminobenzoesäure |
| 0,40 g | Ameisensäure |
| 0,12 g | Cis-1-(3-Chlorallyl)-3,5,7-triaza-1-azoniaadamantanchlorid |

| | |
|---|---|
| 0,10 g | Parfüm |
| 23,00 g | Glycerin(86prozentig) |
| 72,38 g | Wasser |
| 100,00 g | |

**Beispiel 25:** Festigendes Haarstyling-Gel

| | |
|---|---|
| 0,1 g | Polyamidfaser |
| 2,50 g | Polyvinylpyrrolidon |
| 2,00 g | Hydroxypropyl-Guar |
| 0,80 g | Hydriertes Rizinusöl, oxethyliert mit 45 Mol Ethylenoxid |
| 0,60 g | DL-2-Pyrrolidon-5-carbonsäure |
| 0,45 g | Natriumbenzoat |
| 0,30 g | Hydroxyethylcellulose |
| 0,20 g | Parfüm |
| 0,09 g | Natriumformiat |
| 0,05 g | Mica/Titanoxid/Zinnoxid-Pulver (Soloron® Silver Sparkle der Firma Merck/Deutschland) |
| 92,91 g | Wasser |
| 100,00 g | |

**Beispiel 26:** Haarfestigendes Liquid-Gel

| | |
|---|---|
| 0,15 g | Seidenfaser (2) |
| 3,00 g | Vinylpyrrolidon/Vinylacetat Copolymer |
| 1,80 g | Polyoxyethylen-(20)-sorbitanmonopalmitat |
| 1,35 g | Polyethylenglykol-(45) |
| 1,05 g | Hydroxyethylcellulose |
| 1,00 g | Zitronensäure |
| 0,20 g | 1,2-Dibrom-2,4-dicyanobutan |
| 0,20 g | Parfüm |
| 91,25 g | Wasser |
| 100,00 g | |

Sämtliche angegebenen Prozentzahlen stellen Gewichtsprozente dar, sofern nichts anderes vermerkt ist.

**Patentansprüche**

1. Verwendung von mindestens einer wasserunlöslichen synthetischen oder natürlichen Faserart oder deren Gemische zur Herstellung eines kosmetischen Mittels zur Behandlung, Gestaltung oder Pflege der Frisur.

2. Verwendung nach Anspruch 1, dadurch gekennzeichnet, daß die Faserart ausgewählt ist aus der Gruppe bestehend aus Viskose-, Polyester-, Polyamid-, Seiden-, Cellulose-, Flachs-, Leinen-, Schafwoll- oder Baumwollfasern.

3. Verwendung nach einem der Ansprüche 1 bis 2, dadurch gekennzeichnet, daß die Fasern eine Länge von 20 bis 2000 μm und einen Durchmesser von 8 bis 70 μm aufweisen.

4. Verwendung nach einem der Ansprüche 1 bis 2, dadurch gekennzeichnet, daß eine Seidenfaser mit einer Länge von 1 bis 1100 μm verwendet wird.

5. Verwendung nach einem der Ansprüche 1 bis 3, dadurch gekennzeicnnet, daß eine Polyamidfaser mit einer Länge von 150 bis 2000 μm verwendet wird.

6. Verwendung nach einem der Ansprüche 1 bis 5, dadurch gekennzeichnet, daß die Faserart in einer Menge von 0,01 bis 2,5 Gewichtsprozent des kosmetischen Mittels eingesetzt wird.

7. Verwendung nach einem der Ansprüche 1 bis 6, dadurch gekennzeichnet, daß das kosmetische Mittel zusätzlich mindestens ein natürliches oder synthetisches, verdickendes oder haarfestigendes Polymer enthält.

8. Verwendung von mindestens einer wasserunlöslichen synthetischen oder natürlichen Faserart zur Behandlung, Gestaltung oder Pflege der Frisur.